# EUROPÄISCHE PATENTSCHRIFT

(11) **EP 1 716 883 B1**
(45) Veröffentlichungstag und Bekanntmachung des Hinweises auf die Patenterteilung: **27.08.2008**
(21) Anmeldenummer: 06008860.6
(22) Anmeldetag: 28.04.2006
(51) Int. Cl.: A61M 37/00

(54) **Vorrichtung und Set für Implantate**
Device and set for implants
Dispositif et ensemble pour implants

(30) Priorität: 29.04.2005 DE 102005020078
(43) Veröffentlichungstag der Anmeldung: 02.11.2006
(73) Patentinhaber: Novosis AG, 83714 Miesbach (DE)
(72) Erfinder: Bussmann, Oliver, 83229 Aschau (DE); Dabdoub, Nikola, 80933 München (DE)

(56) Entgegenhaltungen:
- NL-A- 9 000 187
- US-A- 1 655 158
- US-A- 3 016 895
- US-A- 5 827 234
- US-B1- 6 450 938

## Beschreibung

Die Erfindung betrifft eine Vorrichtung für eine Implantatkanüle und einen Set mit der erfindungsgemäßen Vorrichtung.

Implantate werden als Stäbchen aus einem biologisch abbaubaren Kunststoff vorgesehen. Derartige Stäbchen können beispielsweise mit einer Pinzette von vorn in eine Kanüle eingeführt werden, die mit einem Kolben versehen ist. Dazu kann eine Lösung des Kunststoffs vorgesehen werden, aus dem das Implantatstäbchen besteht. Mit Hilfe einer Dosierpipette kann dann ein dosierter Tropfen, also eine vordefinierte Menge des gelösten Kunststoffs, in die Kanüle und vor das Implantatstäbchen eingebracht werden. Der Tropfen sitzt dann vor dem Implantatstäbchen und haftet sowohl an der Innenwandung der Kanüle als auch an dem Implantatstäbchen und härtet bei Raumtemperatur aus. Er sichert damit ein Herausfallen des Implantatstäbchens aus der Kanüle. Soll ein Implantatstäbchen appliziert werden, so wird es mit Hilfe des Kanülkolbens gegen den ausgehärteten Kunststofftropfen gepresst, der sich dabei löst und das Implantatstäbchen freigibt. Ein Beispiel für diesen Stand der Technik stellt die Zoladex-Spritze dar; web page "Zoladex-Wirkungsmechanismus".

NL-A-9000187 beschreibt eine Hülse für eine Implantatkanüle, in die eine Kanüle, die mit einem an einem Ring angebrachten Vorsprung versehen ist, eingeführt und mittels Vorsprung und einem quer zur Längsachse federnd angebrachten Arretierungselement festgeklemmt werden kann.

US-A-5,827,234 beschreibt eine Vorrichtung zum Verabreichen von Implantaten, die ein Sicherungselement mit einem Kolbenkanal und einem quer zur Längsachse federnd angebrachten Arretierungselement umfasst, wobei das Arretierungselement eine unbeabsichtigte Bewegung eines Implantats im Kolbenkanal verhindert.

Der geschilderte Stand der Technik ist insofern verbesserungsbedürftig, als mehrere Arbeitsschritte erforderlich sind, um eine Kanüle mit Implantatstäbchen für einen Einsatz vorzubereiten. Ausserdem kann in der Regel eine reproduzierbare Position des Tropfens in der Kanüle nicht gewährleistet werden. Die Oberfläche des Implantatstäbchens wird von Fall zu Fall unregelmäßig vergrößert, zumal das Implantatstäbchen durch die Kunststofflösung angelöst wird. Dies hat negative Folgen bei der Freisetzung des Wirkstoffs aus dem Implantatstäbchen.

Aufgabe der Erfindung ist es, den Stand der Technik zu verbessern.

Die der Erfindung zugrundeliegende Aufgabewird durch eine Vorrichtung gemäß des Anspruchs 1 gelöst .

Die Hülse der erfindungsgemäßen Vorrichtung kann einteilig ausgebildet sein oder aus zwei miteinander verbundenen Halbschalen bestehen.

Ferner kann die Hülse der erfindungsgemäßen Vorrichtung ein Zylinder und der Kanal eine in Längsrichtung des Zylinders verlaufende Lochbohrung sein.

Ferner kann die erfindungsgemäße Vorrichtung als Hülse für eine Implantatkanüle eine Länge aufweisen, die geringer ist als die der Kanüle mit Einstechspitze, Schaft und Kopf.

Ferner kann die erfindungsgemäße Vorrichtung als Hülse eine Länge aufweisen, die der des Schafts einer Kanüle entspricht oder etwa entspricht.

Ferner kann das Arretierungselement der erfindungsgemäßen Vorrichtung eine mit einer Feder vorgespannt Kugel sein.

Ferner kann für die erfindungsgemäße Feder eine Spiralfeder vorgesehen sein.

Ferner kann bei der erfindungsgemäßen Vorrichtung mit Feder in einer Ausnehmung des Kanals sitzen.

Ferner kann das Arretierungselement der erfindungsgemäßen Vorrichtung als Feder mit vorgespannter Kugel in einer Ausnehmung des Kanals sitzen, die als Querbohrung bis in den Kanal niedergebracht ist.

Ferner kann für die erfindungsgemäße Vorrichtung die Ausnehmung mit einer Taille oder einer Verjüngung als Sitz für die in den Kanal vorgespannte Kugel versehen sein.

Ferner kann für die erfindungsgemäße Vorrichtung die Querbohrung einen Sitz für die Feder aufweisen, der vorzugsweise als äußerer Verschluß der Querbohrung ausgebildet ist.

Ferner kann für die erfindungsgemäße Vorrichtung die Kugel an die Feder angeformt sein.

Ferner kann für die erfindungsgemäße Vorrichtung die Feder an den Verschluß angeformt sein.

Ferner kann die erfindungsgemäße Vorrichtung die Hülse aus einem sterilisierbaren Kunststoff bestehen.

Ferner kann das Arretierungselement der erfindungsgemäßen Vorrichtung aus einem sterilisierbaren Kunststoff bestehen.

Ferner kann für die erfindungsgemäße Vorrichtung die Feder aus Edelstahl bestehen.

Eine weitere Ausführungsform der Erfindung betrifft einen Set umfassend mindestens eine erfindungsgemäße Vorrichtung mit Kanüle und fakultativem Implantat, wobei der Schaft der Kanüle mit einer seitlichen Öffnung versehen ist, in die das Arretierungselement vorgespannt ist und durch die das Arretierungselement das Implantat festklemmt.

Der erfindungsgemäße Set kann mit einer Kanüle vorgesehen sein, deren Kopf mit einem Anschlag zum Anschlagen an der Vorrichtung versehen ist.

Nachstehend wird die Erfindung durch drei Figuren beispielhaft erläutert. Es zeigen:
Fig. 1 eine Draufsicht auf die Kopfseite einer erfindungsgemäßen Vorrichtung im Querschnitt,
Fig. 2 eine Draufsicht auf eine erfindungsgemäße Vorrichtung als Schnittansicht von der Seite und
Fig. 3 eine Schnittansicht gemäß Figur 2 mit Kanüle und Implantatstäbchen.

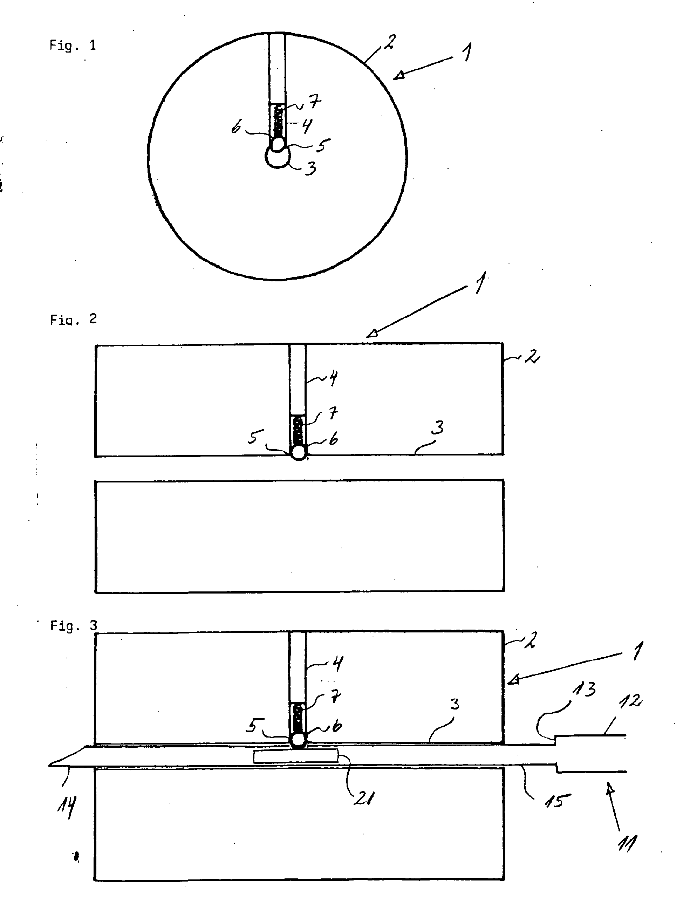

Figur 1 zeigt also eine Draufsicht auf die Kopfseite einer erfindungsgemäßen Vorrichtung 1, und zwar eine Schnittansicht durch die Vorrichtung, die als Zylinder 2 ausgebildet ist. Dabei ist in Längsrichtung durch den Zylinder 2 eine Lochbohrung vorgesehen. Der Querschnitt dieser Lochbohrung 3 ist so bemessen, dass die Bohrung 3 als Kanal eine Implantatkanüle aufnehmen und führen kann. Quer zur Längsbohrung 3 ist eine weitere Bohrung 4 (Querbohrung) bis in den Kanal der Längsbohrung bzw. Lochbohrung 3 niedergebracht. In dieser Querbohrung beziehungsweise in diesem Kanal 4 befindet sich eine Verschlußkugel 6, die beweglich auf einer Verjüngung 5 der Bohrung 4 gelagert ist. Einer Bewegung der Verschlusskugel 6 von der Lochbohrung 3 weg wirkt die Kraft einer in die Bohrung 4 eingesetzten Spiralfeder 7 entgegen. Je nach Stabilität eines zu sichernden Implantates kann der benötigte Druck der Verschlußkugel 6 auf ein in die Lochbohrung 3 eingeführtes Implantat durch verschieden harte Spiralfedern 7 voreingestellt werden.

Figur 2 zeigt eine in Längsrichtung geschnittene Vorrichtung 1. Man erkennt die Lochbohrung 3, deren Durchmesser einer Kanüle entspricht, ferner die quer auf die Längsbohrung 3 niedergebrachte Bohrung 4, die Verschlußkugel 6 und die Spiralfeder 7. Die Verschlußkugel 6 wird durch eine bereits angesprochene Verjüngung 5 am unteren Ende der Bohrung 4 gegen ein Herausfallen der Kugel 6 in die Längsbohrung 3 gesichert.

Die erfindungsgemäße Vorrichtung 1 kann also als Zylinder 2 vorgesehen sein. Durch den Zylinder 2 ist in Längsrichtung eine Lochbohrung 3 eingebracht, die dem Aussendurchmesser einer Implantatkanüle entspricht. Quer zur Lochbohrung 3 ist eine weitere Bohrung 4 vorgesehen, die in die Lochbohrung 3 mündet. Diese Bohrung 4 verjüngt sich geringfügig zur Lochbohrung 3. In der Bohrung 4 ist eine Kugel 6 als Verschlußkugel vorgesehen. Die Kugel wird mit Hilfe einer Spiralfeder 7 gegen die Lochbohrung 3 vorgespannt.

Figur 3 zeigt eine Implantatkanüle 11, die in die Lochbohrung 3 einer erfindungsgemäßen Vorrichtung 1 eingeführt ist. Die Implantatkanüle 11 ist mit einer seitlichen Öffnung bzw. Perforation 16 versehen, durch die die Verschlußkugel 6 in das Rohr bzw. in den Kanal 15 der Implantatkanüle 11 hineinragt. Damit werden Kanüle 11 und Implantat 21 mit einer vordefinierten Kraft festgeklemmt. Wenn man die Vorrichtung 1 relativ zur Implantatkanüle 11 dreht, wird die Verschlußkugel 6 über den Rand der in der Kanüle 11 vorgesehenen Öffnung 16 angehoben, wodurch die Verschlußkugel 6 das Implantat 21 freigibt. Das Implantat 21 kann nun mit Hilfe eines nicht dargestellten Kolbens aus der Kanüle 11 ausgestoßen und implantiert werden.

Die Vorrichtung 1 kann in entriegelter Position auf der Implantatkanüle 11 verbleiben und nach erfolgter Implantation mit der Kanüle 11 entsorgt werden.

### Bezugszeichenliste

- 1: Erfindungsgemäße Vorrichtung (Implantatklammer)
- 2: Zylinder
- 3: Lochbohrung
- 4: (Quer)-Bohrung
- 5: Verjüngung
- 6: Verschlußkugel
- 7: Spiralfeder

- 11: Implantatkanüle
- 12: Kopf der Kanüle
- 13: Anschlag
- 14: Applikationsspitze der Kanüle
- 15: Kanülenrohr

- 21: Implantat

## Patentansprüche

1. Vorrichtung (1) in Form einer Hülse für eine Implantatkanüle (11) mit Implantat (21), wobei die Hülse aufweist: einen in Längsrichtung der Hülse verlaufenden Kanal, in den eine Implantatkanüle (11) mit Implantat (21) eingeführt werden kann, sowie ein in den Kanal quer zu seiner Längsachse federnd vorgespanntes Arretierungselement, **dadurch gekennzeichnet, daß** das Arretierungselement eingerichtet ist, um Kanüle und Implantat festzuklemmen.

2. Vorrichtung (1) nach Anspruch 1, wobei die Hülse einteilig ausgebildet ist oder aus zwei miteinander verbundenen Halbschalen besteht.

3. Vorrichtung (1) nach Anspruch 1 und/oder 2, wobei die Hülse ein Zylinder (2) und der Kanal eine in Längsrichtung des Zylinders (2) verlaufende Lochbohrung (3) ist.

4. Vorrichtung (1) nach mindestens einem der vorhergehenden Ansprüche, wobei sie als Hülse für eine Implantatkanüle (11) eine Länge aufweist, die geringer ist als die der Kanüle mit Einstechspitze, Schaft und Kopf.

5. Vorrichtung (1) nach Anspruch 4, wobei sie als Hülse eine Länge aufweist, die der des Schafts einer Kanüle entspricht oder etwa entspricht.

6. Vorrichtung (1) nach mindestens einem der vorhergehenden Ansprüche, wobei das Arretierungselement eine mit einer Feder vorgespannte Kugel (6) ist.

7. Vorrichtung (1) nach Anspruch 6, wobei die Feder eine Spiralfeder (7) ist.

8. Vorrichtung (1) nach Anspruch 6 und/oder 7, wobei Kugel (6) mit Feder in einer Ausnehmung des Kanals sitzt.

9. Vorrichtung (1) nach mindestens einem der Ansprüche 6 bis 8, wobei das Arretierungselement als Feder mit vorgespannter Kugel (6) in einer Ausnehmung des Kanals sitzt, die als Querbohrung (4) bis in den Kanal niedergebracht ist.

10. Vorrichtung (1) nach Anspruche 8 und/oder 9, wobei die Ausnehmung mit einer Taille oder einer Verjüngung (5) als Sitz für die in den Kanal vorgespannte Kugel (6) versehen ist.

11. Vorrichtung (1) nach mindestens einem der Ansprüche 9 bis 10, wobei die Querbohrung (4) einen Sitz für die Feder aufweist, der vorzugsweise als äußerer Verschluß der Querbohrung (4) ausgebildet ist.

12. Vorrichtung (1) nach mindestens einem der Ansprüche 6 bis 11, wobei die Kugel (6) an die Feder angeformt ist.

13. Vorrichtung (1) nach mindestens einem der Ansprüche 11 und/oder 12, wobei die Feder an den Verschluß angeformt ist.

14. Vorrichtung (1) nach mindestens einem der vorhergehenden Ansprüche, wobei die Hülse aus einem sterilisierbaren Kunststoff besteht.

15. Vorrichtung (1) nach mindestens einem der vorhergehenden Ansprüche, wobei das Arretierungselement aus einem sterilisierbaren Kunststoff besteht.

16. Vorrichtung (1) nach mindestens einem der Ansprüche 6 bis 14, wobei die Feder aus Edelstahl besteht.

17. Set umfassend mindestens eine Vorrichtung (1) gemäß einem der vorhergehenden Ansprüche mit Kanüle (11) und fakultativem Implantat, (21) wobei der Schaft der Kanüle (11) mit einer seitlichen Öffnung versehen ist, in die das Arretierungselement vorgespannt ist und durch die das Arretierungselement das Implantat (21) festklemmt.

18. Set nach Anspruche 17 mit einer Kanüle (11), deren Kopf (12) mit einem Anschlag (13) zum Anschlage an der vorrichtung versehen ist.

## Claims

1. Apparatus (1) in the form of a sleeve for an implant cannula (11) with an implant (21), wherein the sleeve comprises: a channel oriented in longitudinal direction of the sleeve, in which channel an implant cannula (11) with an implant (21) can be introduced, as well as an arrest element that is resiliently pretensioned into the channel across the longitudinal axis thereof, **characterized in that** the arrest element is adjusted to lock the cannula (11) and the implant (21).

2. Apparatus (1) according to claim 1, wherein the sleeve is formed as a single part or consists of two half-shells joined with each other.

3. Apparatus (1) according to claim 1 and/or 2, wherein the sleeve is a cylinder (2), and the channel is a bore hole (3) oriented in longitudinal direction of the cylinder (2).

4. Apparatus (1) according to at least one of the preceding claims, wherein the apparatus (1), as a sleeve for an implant cannula (11), has a length which is smaller than that of the cannula (11) with injection tip, shaft and head.

5. Apparatus (1) according to claim 4, wherein the apparatus (1), as a sleeve, has a length which corresponds or approximately corresponds to that of the shaft of a cannula.

6. Apparatus (1) according to at least one of the preceding claims, wherein the arrest element is a ball (6) pretensioned with a spring.

7. Apparatus (1) according to claim 6, wherein the spring is a coil spring (9).

8. Apparatus (1) according to claim 6 and/or 7, wherein the ball (6) is located in a recess of the channel.

9. Apparatus (1) according to at least one of the claims 6 to 8, wherein the arrest element, as a spring with a pretensioned ball (6), is located in a recess of the channel which recess is formed as a cross hole (4) down into the channel.

10. Apparatus (1) according to claim 8 and/or 9, wherein the recess is provided with a waist or a diminution (5) as a socket for the ball (6) pretensioned into the channel.

11. Apparatus (1) according to at least one of the claims 9 to 10, wherein the cross hole (4) has a socket for the spring which socket is formed preferably as an exterior closure of the cross hole (4).

12. Apparatus (1) according to at least one of the claims 6 to 11, wherein the ball (6) is formed in combination with the spring.

13. Apparatus (1) according to at least one of the claims 11 and/or 12, wherein the spring is formed in combination with the closure.

14. Apparatus (1) according to at least one of the preceding claims, wherein the sleeve consists of a synthetic material capable of sterilization.

15. Apparatus (1) according to at least one of the preceding claims, wherein the arrest element consists of a synthetic material capable of sterilization.

16. Apparatus (1) according to at least one of the claims 6 to 14, wherein the spring consists of stainless steel.

17. Set comprising at least one apparatus (1) according to one of the preceding claims with a cannula (11) and an optional implant (21), wherein the shaft of the cannula (11) is provided with a lateral hole into which the arrest element is pretensioned and through which the arrest element locks the implant (21).

18. Set according to claim 17 with a cannula (11), the head (12) of which is provided with an end stop (13) for stopping at the apparatus (1).

## Revendications

1. Dispositif (1) sous forme d'un manchon pour une canule d'implant (11) avec implant (21), dans lequel le manchon comprend : un canal s'étendant dans la direction longitudinale du manchon dans lequel une canule d'implant (11) avec implant (21) peut être introduite, ainsi qu'un élément d'arrêtage précontraint de manière flexible dans le canal perpendiculairement à son axe longitudinal, **caractérisé en ce que** l'élément d'arrêtage est installé de manière à bloquer la canule et l'implant.

2. Dispositif (1) selon la revendication 1, dans lequel le manchon est conçu en une seule partie ou est constitué de deux demi-coquilles reliées l'une à l'autre.

3. Dispositif (1) selon la revendication 1 et/ou 2, dans lequel le manchon est un cylindre (2) et le canal est un sondage (3) s'étendant dans la direction longitudinale du cylindre (2).

4. Dispositif (1) selon au moins l'une quelconque des revendications précédentes, dans lequel, sous forme de manchon pour une canule d'implant (11), il présente une longueur qui est inférieure à celle de la canule avec pointe d'incision, tige et tête.

5. Dispositif (1) selon la revendication 4, dans lequel, sous forme de manchon, il présente une longueur qui correspond ou qui correspond à peu près à celle de la tige d'une canule.

6. Dispositif (1) selon au moins l'une quelconque des revendications précédentes, dans lequel l'élément d'arrêtage est une bille (6) précontrainte avec un ressort.

7. Dispositif (1) selon la revendication 6, dans lequel le ressort est un ressort en spirale (7).

8. Dispositif (1) selon la revendication 6 et/ou 7, dans lequel la bille (6) avec ressort est positionnée dans un évidement du canal.

9. Dispositif (1) selon au moins l'une quelconque des revendications 6 à 8, dans lequel l'élément d'arrêtage est positionné sous forme de ressort avec bille précontrainte (6) dans un évidement du canal qui est foré comme un alésage transversal (4) jusque dans le canal.

10. Dispositif (1) selon la revendication 8 et/ou 9, dans lequel l'évidement est muni d'un rétrécissement ou d'un étranglement (5) servant de siège pour la bille (6) précontrainte dans le canal.

11. Dispositif (1) selon au moins l'une quelconque des revendications 9 à 10, dans lequel l'alésage transversal (4) comporte un siège pour le ressort qui est conçu, de préférence, comme fermeture extérieure de l'alésage transversal (4).

12. Dispositif (1) selon au moins l'une quelconque des revendications 6 à 11, dans lequel la bille (6) est moulée sur le ressort.

13. Dispositif (1) selon au moins l'une quelconque des revendications 11 et/ou 12, dans lequel le ressort est moulé sur la fermeture.

14. Dispositif (1) selon au moins l'une quelconque des revendications précédentes, dans lequel le manchon est constitué d'une matière plastique stérilisable.

15. Dispositif (1) selon au moins l'une quelconque des revendications précédentes, dans lequel l'élément d'arrêtage est constitué d'une matière plastique stérilisable.

16. Dispositif (1) selon au moins l'une quelconque des revendications 6 à 14, dans lequel le ressort est fait en acier spécial.

17. Set comprenant au moins un dispositif (1) selon au moins l'une quelconque des revendications précédentes avec canule (11) et implant facultatif (21), dans lequel la tige de la canule (11) est munie d'une ouverture latérale dans laquelle l'élément d'arrêtage est précontraint et au moyen de laquelle l'élément d'arrêtage bloque l'implant (21).

18. Set selon la revendication 17 avec une canule (11) dont la tête (12) est munie d'une butée (13) pour venir en butée sur le dispositif.
